# EUROPEAN PATENT SPECIFICATION

(11) **EP 1 738 747 B1**
(45) Date of publication and mention of the grant of the patent: **02.01.2008**
(21) Application number: 05014134.0
(22) Date of filing: 30.06.2005
(51) Int. Cl.: A61K 8/68, A61Q 19/00, A61Q 19/02, A61Q 19/06, A61Q 19/08

(54) **Use of salicyloyl-sphingoidbase for the treatment or prevention of cellulite**
Verwendung von Salicyloyl-Sphingoidbase zur Behandlung oder Verhinderung von Cellilitis
Utilisation d'une base salicyloyl-sphingoide pour le traitement ou prévention de la cellulite

(43) Date of publication of application: 03.01.2007
(73) Proprietor: Evonik Goldschmidt GmbH, 45127 Essen (DE)
(72) Inventor: Farwick, Mike Dr., 45130 Essen (DE); Rawlings, Anthony, Vincent, Northwich Cheshire CW9 8FH (GB)

(56) References cited:
- EP-B- 0 966 431
- US-A- 5 578 641
- US-A- 5 705 170
- US-A- 5 882 665
- US-A- 6 147 118
- ANONYMOUS: "Phytosphingosine SLC" GOLDSMIDT PERSONAL CARE, [Online] XP002351686 Retrieved from the Internet: URL:http://web.archive.org/web/20040521231 432/http://centerchem.com/PDFs/Phytosphing osine_SLC.pdf> [retrieved on 2004-05-21]

## Description

### Field of the invention

This invention relates to the use of topical compositions containing salicyloyl-sphingoidbases for application to human skin for the treatment and/or prevention of cellulite.

### Background and prior art

Skin is subject to deterioration through the passage of time (chronological aging, dermatological disorders, hormonal changes, environmental abuse (wind, air conditioning, central heating, pollution) all of which may be accelerated by exposure of skin to radiation from the sun (photoaging). Consumers are increasingly seeking "anti-aging" cosmetic products which treat or delay the visible signs of chronological aging and photoaging skin such as wrinkles, lines, sagging, hyperpigmentation and age spots. Cellulite also manifests itself as a result of increased adipogenicity and reduced connective tissue matrix.

Skin care cosmetic and dermatological compositions for improving the condition and appearance of skin comprising sphingolipids operate mainly in the epidermal layers of the skin.

There continues to be a need, however, for alternative effective cosmetic compositions for topical application to skin for treating/delaying cellulite.

Sphingolipid derivatives and ceramides etc have previously used to treat the barrier of the skin (i. e. the epidermis) we have now surprisingly found that effective treatment and prevention of normal skin conditions due to cellulite may be obtained through the application of cosmetic compositions to the skin which comprise a specific sphingolipid: salicyloyl-phytosphingosine, acting primarily to increase dermal proteins.

### Summary of the invention

According to a first aspect of the present invention there is provided a topical composition comprising:
(a) salicyloyl-phytosphingosine and
(b) a dermatologically acceptable carrier.

Such compositions are particularly useful for topical application to human skin especially for the treatment and/or prevention of cellulite.

The use of these compositions provides anti-cellulite benefits which result in the promotion of smooth and supple skin with improved elasticity and a reduced or delayed and/or orange-peel skin. A general improvement in the appearance, texture and condition, in particular with respect to the radiance, clarity, firmness, smoothness and general youthful appearance of skin is achieved.

The term "treating" as used herein includes within its scope reducing, delaying and/or preventing, within its scope reducing, delaying and/or preventing cellulitic and generally enhancing the quality of skin and improving its appearance and texture by preventing and increasing flexibility, suppleness and elasticity of the skin. The cosmetic compositions, methods and the uses of the salicyloyl-phytosphingosine according to the invention may be useful for treating skin which is already in a cellulitic condition or for treating youthful skin to prevent or reduce those aforementioned deteriorative changes due to the normal aging process.

### Detailed Description of the Invention

Details of structure and/or derivatives thereof.

The structure of Salicyloyl-Phytosphingosine is shown below:

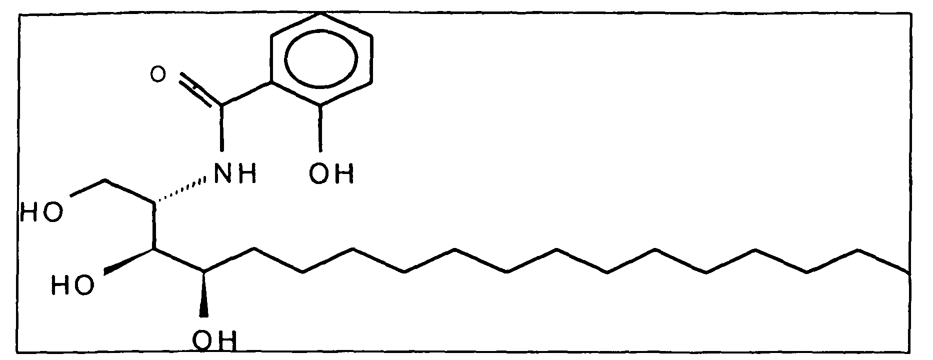

The invention also includes the use of other derivatives. Preferable derivatives include derivatives of the hydroxyl groups of the phytosphingosine (PS) moiety, such as organic and inorganic esters e. g. phosphate esters, sulphate esters and acetate esters or glycosylated derivatives. The invention also includes other salicyloyl-sphingoidbase derivatives, e. g. salicyloyl-sphingosine, salicyloyl-sphinganine, salicyloyl-6-hydroxy-sphinganine.

A preferred method of preparation is disclosed in Example 1 below.

The active, salicyloyl-phytosphingosine, to be employed in accordance with the present invention is present in the topical composition in an effective amount. Normally the total amount of the active is present in an amount between 0,001 and 20 % by weight of the composition. More preferably the amount is from 0,01 to 10 % and most preferably from 0,02 to 2 % in order to maximize benefits at a minimum cost.

### Optional Skin Benefit Materials and Cosmetic Adjuncts

Besides the active, salicyloyl-phytosphingosine, other specific skin-benefit actives such as sunscreens, skin lightening agents, skin tanning agents may also be included. The vehicle may also further include adjuncts such as perfumes, antioxidants, opacifiers, preservatives, colourants and buffers.

Typical additional bioactive compounds are tocopherol and derivatives, ascorbic acid and derivatives, desoxyribonucleic acid, retinol and derivatives, caffeine and derivatives, alpha-lipoic acid, bisabolol, allantoin, phytantriol, panthenol, alpha hydroxy acids, amino acids, hyaluronic acid and derivatives, creatine and derivatives, trimethylglycine, myoinositol, pyroglutamatic acid and derivatives, taurine, guanidine and derivatives, phospholipids, lysophospholipids, ceramides, phytosphingosine and derivatives, sphingosine and derivatives, pseudoceramides and derivatives, essentiell oils, peptides, modified peptides, proteinhydrolysates, plant extracts and vitamin complexes.

Beneath in skin care applications as decribed above, salicyloyl-phytosphingosine can also be incorporated into hair care formulations like shampoos and conditioners, where it has a stimulating effect on the scalp performance.

### Dermatologically Acceptable Vehicle

The composition according to the invention also comprises a dermatologically/cosmetically acceptable vehicle to act as a dilutant, dispersant or carrier for the active, salicyoyl-phytosphingosine. The vehicle may comprise materials commonly employed in skin care products such as water, liquid or solid emollients, silicone oils, emulsifiers, solvents, humectants, thickeners, powders, propellants and the like. Other agents can be:
Sphingoid and phospholipids derivatives: ceramides, phytosphingosine, sphingosine, pseudoceramides, phospholipids, lysophospholipids
Antioxidants and vitamins: tocopherol and derivatives, ascorbic acid and derivatives, niacinamide and derivatives, vitamin complexes, alpha-lipoic acid, retinol and derivatives, panthenol
Antiinflammatories: bisabolol, allantoin, phytantriol, Coenzyme Q10 Idebenone.

Botanical agents such as polyphenolics, flavonoids or isoflavones.

Moisturising agents: amino acids, hyaluronic acid and derivatives, creatine and derivatives, trimethylglycine, myoinositol, pyroglutamatic acid and derivatives, taurine, guanidine and derivatives and hydroxy acids.

Skin whitening agents: kojic acid, arbutin, vitamin C and derivatives, hydroquinone

Peptides, modified peptides, proteinhydrolysates

Caffeine

Sunscreens and UV-Absorbers

The vehicle will usually form from 80 to 99,99 %, preferably from 90 to 99,99 % and most preferably from 99,98 to 98 % by weight of the composition, and can, in the absence of other cosmetic adjuncts, form the balance of the composition.

The skin care formulation can be an aqueous solution, water-in-oil (w/o) emulsion, oil-in-water (o/w) emulsion, a dispersion of lipids, an aqueous, water-alcohol, oil or oil-alcohol gel, a solid stick, a wet-wipe or an aerosol.

If the vehicle itself is an (w/o) or (o/w) emulsion, it contains 5 to 50 % of an oilphase and 47 to 94,95 % water, with respect to the weight of the whole formulation.

Typical guideline formulations are public available and can be obtained by the manufacturers of either the raw materials or the active ingredients (e. g.: Kosmetik-Jahrbuch, B. Ziolkowsky, Verlag für Chemische Industrie, Germany). The cosmetic formulations show an anti-cellulite effect.

### Product Preparation, Form, Use and Packaging

To prepare the topical composition according to the present invention the usual manner for preparing skin care products may be employed. The active components are generally incorporated in a dermatologically acceptable carrier in conventional manner. The active components can suitably first be dissolved or dispersed in a portion of the water or another solvent or liquid to be incorporated in the composition. The preferred compositions are oil-in-water or water-in-oil emulsions.

The composition may be in the form of conventional skin-care products such as a cream, gel or lotion or the like. The composition can also be in the form of a so-called "wash-off" product e. g. a bath or shower gel, possibly containing a delivery system for the actives to promote adherence to the skin during rinsing. Most preferably the product is a "leave-on" product; a product to be applied to the skin without a deliberate rinsing step soon after its application to the skin.

The composition may be packaged in any suitable manner such as in a jar, a bottle, tube, roll-ball, or the like, in the conventional manner.

The active ingredients described in the present invention may be applied one or more times daily to the skin which requires treatment. The improvement in skin appearance will usually become visible after 1 to 6 months, depending on skin condition, the concentration of the active components used in the inventive method, the amount of composition used and the frequency with which it is applied. In general, a small quantity of the composition, for example from 0,1 to 5 ml is applied to the skin from a suitable container or applicator and spread over and/or rubbed into the skin using the hands or fingers or a suitable device. A rinsing step may optionally follow depending on whether the composition is formulated as a "leave-on" or a "rinse-off" product.

In order that the present invention may be more readily understood, the following examples are given, by way of illustration only.

Many studies have shown that the levels of collagen type I in skin is decreased with age Collagen, the predominant matrix skin protein is known to impart tensile strength to skin. Fibrillin is an important protein acting at the dermoepidermal junction of the skin. It is also known in the art that the levels of collagen and fibrillin in skin are significantly reduced with aged (for example: Lavker, R. J. Inv. Derm.(1979), 73, 79 - 66; Griffiths et al. N. Eng. J. med. (1993), 329, 530 - 535). The reduction of the levels of these skin proteins is accordingly associated with a decrease in the tensile strength of the skin As a result of this laxity cellulite is more visible.

It is well known in the art that retinoic acid is a potent active. It has been shown that dermal repair following topical treatment of skin with retinoic acid arises through new collagen deposition and synthesis in the skin (for example: Griffiths et al, N. Eng. J. med. (1993), 329, 530 - 535).

Procollagen I is a precursor of collagen. Increased production of procollagen I in response to a test compound application is a marker of an increased collagen level. Matrix-Metallo-Proteases (MMP) are collagen degrading enzymes. Their decreased expression will lead to less collagen degradation and thus to an increased collagen level.

### EXAMPLES

### Example 1 ANTI-AGEING USE NOT CLAIMED

This example demonstrates the anti-ageing benefits of a salicyoyl-phytosphingosine (PS-SLC)

It was done by identification of Procollagen-I upregulation and matrix metalloprotease-1 (MMP-1) down-regulation in Skin In Vivo. Experiments were done following topical Retinoic Acid (RA) treatment for comparative purposes and analysis of the effects of salicyloyl-phytosphingosine compared with retinoic acid with respect to the expression of dermal markers in vivo.
See: Watson REB, Craven NM, Kang S, Jones CJP, Kielty CM, Griffiths CEM. A short term screening protocol, using fibrillin-1 as a receptor molecule for photoageing repair agents. J Invest Dermatol, 116: 672 - 678, 2001, for published methodology.

Patch-test protocol and quantification of markers
Five healthy volunteers were recruited (age range 54 - 71 years). Test substances were applied separately under standard 6 mm diameter Finn chambers to the extensor aspect of the forearm: these were vehicle base(15 µl/chamber), 0,2 % PS-SLC (15 µl/chamber), 0,05% PS-SLC (15 µl/chamber), and 0,025 % all-trans RA (Retin-A® cream, Janssen-Cilag Ltd., 15 µl/chamber).

Formulations were applied to clean skin on days 1 and 4 of the assay. All-trans RA was applied to an untreated site on day 4. On day 8, Finn chambers were removed and 3 mm punch biopsies were obtained under 1 % lignocaine anaesthesia from each of the test site. Biopsies were embedded in OCT compound (Tissue-Tek®, Miles, IN, USA) and snap frozen in liquid nitrogen. Biopsy sites were sutured with 1 x 4/o ethilon and subjects instructed to return between 7 - 10 days for suture removal. Frozen sections were prepared at a thickness of 10 µm (OTF cryostat, Bright Instruments Ltd.) and mounted onto gelatin-coaled slides prior to histological analysis A number of extracellular matrix (ECM) molecules known to be altered in photoaged skin were assayed by immunohistochemistry to detail the potential effects of the formulations. The primary marker of outcome was the distribution of fibrillin-rich microfibrils proximal to the dermal-epidermal junction (DEJ). Also assessed were the distribution of procollagen-1 within the dermis.

Matrix metalloprotease-1 (MMP-1) levels were also assessed. Whilst the and procollagen-1 should increase room antiaging active levels of MMP 1 should decrease an antiaging active increased synthesis of dermal structural proteina together with reduced levels of the proteases that degrade them. For each analysis, the marker was identified in each of three sections (i. e., 3 sections/treatment/volunteer). Sections were optimally fixed. Routinely, following hydration in tris-buffered saline (TBS; 100 mM Tris, 150 mM NaCl), sections were solublised by addition of 0,5 % Triton-X100 (10 minutes). Following washing, endogenous peroxidase activity was abolished by incubation with an excess of hydrogen peroxide in methanol (30 minutes). Sections were blocked prior to application of primary antibody (overnight incubation @4 °C). Negative controls were concurrently incubated with either block alone or control serum. Following incubation, sections were stringently washed with TBS prior to application of an appropriate biotinylated secondary antibody. This was further conjugated to the enzyme horseradish peroxidase using a commercially available kit following the manufacturers instructions (ABC Elite System, Vector Laboratory, Peterborough UK). Antibody was localised using Vector SG® as chromogen (10 minute incubation) - washing in TBS quenched this reaction. Sections were counterstained using Nuclear Fast Red and finally dehydrated through serial alcohols, cleared and permanently mounted.

| Marker | Host | Clone | Fixation | Dilution |
|---|---|---|---|---|
| Fibrillin-rich microfibrils | Mouse | NeoMarkers; 11C1.3 | 4 % Parafomaldehyde | 1:100 |
| Pro-collagen I | Rat | Chemicon International; M-58 | 4 % Parafomaldehyde | 1:1000 |
| Matrix-Metalloprotease I | Mouse | Abcam, ab2461 | 4 % Parafomaldehyde | 1:100 |

Sections were randomised, blinded and examined on a Nikon OPTIPHOT microscope (Tokyo, Japan). For assessment of ECM and cornified envelope components, the degree of immunostaining was assessed on a 5 point semi-quantitative scale where 0 = no staining and 4 = maximal staining. Four sections (including control) were examined per subject per site. The degree of immunostaining was scored for three high power fields per section, and the average score calculated for each site/test area. Differences in the distribution between the test sites, and after application of test substances for varying periods of time, were assessed for significance using the repeated measures analysis of variance test (ANOVA). To assess whether age or baseline values affected outcome measures, data was tested using paired Student's t-tests. Both models were tested using SPSS+ software (v11.5, SPSS Inc., IL USA) with significance taken at the 95 or at the 80 % confidence level.

### Results

### Erythema

All volunteers tolerated the patch test protocol well. Furthermore, all-trans RA produced marked erythema at the site of application.

### Fibrillin-1

Application of all-trans RA (the "gold" standard) produced deposition of fibrillin-1 proximal to the DEJ in the volunteers. Application of 0,2 % PS-SLC resulted in significantly increased fibrillin-1 deposition in the exact same volunteers using the 95 % confidence level. Application of the 80 % confidence level also showed significant induction of fibrillin expression by 0,05 % PS-SLC and 0,2 % PS.

Figure 1: Effect of formulation on fibrillin expression proximal to the DEJ.

**Descriptive Statistics procollagen I IHC**

| | N | Minimum | Maximum | Mean | Std. Deviation |
|---|---|---|---|---|---|
| Vehicle | 5 | 0,00 | 1,83 | 1,2778 | 0,74536 |
| 0,2% PS-SLC | 5 | 1,33 | 3,22 | 2,267* | 0,83157 |
| 0,05% PS-SLC | 5 | 1,56 | 2,17 | 1,7778^{**} | 0,23895 |
| 02^{%} PS | 5 | 1,22 | 2,67 | 2,0000^{**} | 0,64310 |
| 0,025% RA | 5 | 2,14 | 3,56 | 2,8625^{**} | 0,53309 |
| Valid N (listwise) | 5 | | | | |

| | | | | | |
|---|---|---|---|---|---|
| *p < 0,05, **p < 0,2 | | | | | |

### Pro-collagen I (pCI)

Application of all-trans RA had little effect on the deposition of pCI proximal to the DEJ following 4-day occluded application, in agreement with previous short term studies but it is known to increase levels in the longer term. However, 0,2 % PS-SLC significantly increased pCI content in the papillary dermis using the 95 or the 80 % confidence level, respectively.

**Descriptive Statistics: procollagen I IHC**

| | N | Minimum | Maximum | Mean | Std. Deviation |
|---|---|---|---|---|---|
| Vehicle | 5 | 1,25 | 3,22 | 2,1389 | 0,82543 |
| 0,2% PS-5LC | 5 | 2,11 | 3,56 | 2,7790* | 0,52180 |
| 0,05% PS-SLC | 5 | 1,89 | 2,78 | 2,2472 | 0,33570 |
| | | | | | |
| 0,025% RA | 5 | 1,56 | 2,89 | 2,2667 | 0,59701 |
| Valid N (listwise) | 5 | | | | |

| | | | | | |
|---|---|---|---|---|---|
| *p < 0,05, **p < 0,2 | | | | | |

### Matrix-Metalloprotease-1

Application of all-trans RA had no effect on the expression of MMP-1 following 4-day occluded application, in agreement with previous short term studies. However, for this marker both 0,05 and 0,2 % concentrations of salicyloyl-phytosphingosine decreased MMP-1 levels significantly using the 95 % confidence level.

Figure 3: Effect of novel formulation on MMP1 expression.

**Descriptive Statistics MMPI IHC**

| | N | Minimum | Maximum | Mean | Std. Deviation |
|---|---|---|---|---|---|
| Vehicle | 5 | 11,33 | 43,178 | 33,95 | 13,01 |
| 0,2% PS-SLC | 5 | 13,67 | 31,33 | 22,72* | 10,47 |
| 0,05% PS-SLC | 5 | 0,00 | 37,33 | 18,40* | 13,33 |
| | | | | | |
| 0,025% RA | 5 | 11,54 | 62,75 | 36,82 | 16,01 |
| Valid N (listwise) | 5 | | | | |

| | | | | | |
|---|---|---|---|---|---|
| *p < 0,05 | | | | | |

From this study it was shown that that topical occluded application of PS-SLC restores the fibrillin-rich microfibrillar network proximal to the dermal-epidermal junction of photoaged skin. In addition, PS-SLC also shows beneficial effects on procollagen I in the papillary dermis. Additionally application of salicyloyl-phytosphingosine decreased MMP-1 levels significantly.

All of these effects will be useful for treating cellulitic skin conditions. These are the first ever results demonstrating the effects of these molecules on increasing dermal and dermoepidermal junctional molecules in skin. Sphingolipids have only been shown in the past to treat the barrier properties and the differentiation process of the skin.

### Example 2

The vehicle will usually form from 80 to 99,99 %, preferably from 90 to 99,99 % and most preferably from 99,98 to 98 % by weight of the composition, and can, in the absence of other cosmetic adjuncts, form the balance of the composition.

The skin care formulation can be an aqueous solution, water-in-oil (w/o) emulsion, oil-in-water (o/w) emulsion, a dispersion of lipids, an aqueous, water-alcohol, oil or oil-alcohol gel, a solid stick, a wet-wipe or an aerosol.

If the vehicle itself is an (w/o) or (o/w) emulsion, it contains 5 to 50 % of an oilphase and 47 to 94,95 % water, with respect to the weight of the whole formulation.
The following table shows an expample of a typical o/w formulation:

| | | | |
|---|---|---|---|
| A) | TEGO® Care PS | Methyl Glucose Sesquistearate | 3,0 % |
| | TEGO® Alkanol 18 | Stearyl Alcohol | 1,5 % |
| | TEGIN® M | Glyceryl Stearate | 3,5 % |
| | TEGOSOFT® liquid | Cetearyl Ethylhexanoate | 9,2 % |
| | TEGOSOFT® CT | Caprylic/Capric Triglyceride | 10,0 % |
| | | | |
| B) | Glycerin | | 5,0 % |
| | Water | | 65,45 % |
| | | | |
| C) | TEGO® Carbomer 134 | Carbomer | 0,2 % |
| | TEGOSOF7® liquid | Cetearyl-ethylhexanoate | 0,8 % |
| | | | |
| | Photosphingosine or Salicyloyl-Phytosphingosine | | 0,2 % |
| | NaOH (10%) | | 0,65 % |
| | Perfume | | 0,5 % |
| | Preservative | | q. s. |

### Preparation of the formulation:

Phase A) and B) are heated up to 65 °C. Phase B) is added to A). The mixture is homogenized (e. g. using an Ultra Turrax) and cooled down under slow stirring. At 60 °C phase C) is added, followed by a short homogenization step. NaOH and the Perfume are added at 35 - 40 °C.

## Claims

1. Use of a cosmetic composition comprising:
a) Salicyloyl-sphingoidbases and/or derivatives therof , and
b) a dermatologically acceptable carrier
for treating and/or preventing cellulitic skin by increasing dermal structural proteins (collagen and fibrillin) and reducing the levels of MMP-1.

2. Use of a cosmetic composition according to claim 1 where the Salicyloyl-sphingoidbases is salicyloyl-phytosphingosine.

3. Use of a cosmetic composition according to claim 1 where the Salicyloyl-sphingoidbases is salicyloyl-sphingosine, salicyloyl-sphinganine or salicyloyl-6-hydroxysphinganine.

4. Use of a cosmetic composition according to claim 1-3 where one or more of the hydroxyl groups of the sphingoidbase-moiety of the salicyloyl-sphingoidbase is modified as organic and inorganic esters.

5. Use of a cosmetic composition according to claim 1 - 3 where one or more of the hydroxyl groups of the sphingoidbase-moiety of the salicyloyl-sphingoidbase is modified by glycosylation.

6. Use of a composition according to claim 1 - 5 comprising 0,001 to 20 %, preferably 0,01 to 10 % by weight and most preferably 0,02 - 2 % by weight of the composition of said salicyloyl-sphingoidbase, sphingoidbase and/or derivatives.

7. Use of a topical composition according to claims 1 - 6 in combination with at least one compound selected from the group consisting of
a) Sphingoid and phospholipids derivatives: ceramides, phytosphingosine, sphingosine, pseudoceramides, phospholipids, lysophospholipids,
b) Antioxidants and vitamins such as tocopherol and derivatives, ascorbic acid and derivatives, niacinamide and derivatives, vitamin complexes, alpha-lipoic acid, retinol and derivatives, panthenol,
c) Antiinflammatories such as bisabolol, allantoin, phytantriol, Coenzyme Q10, Idebenone,
d) Botanical agents such as polyphenolics, flavonoids and isoflavones,
e) Moisturising agents such as amino acids, hyaluronic acid and derivatives, creatine and derivatives, trimethylglycine, myoinositol, pyroglutamatic acid and derivatives, taurine, guanidine and derivatives and hydroxy acids,
f) Skin whitening agents such as kojic acid, arbutin, vitamin C and derivatives, hydroquinone,
g) Peptides, modified peptides, proteinhydrolysates,
h) Caffeine,
i) Sunscreens and UV-Absorbers.

## Patentansprüche

1. Verwendung einer Kosmetik-Zusammensetzung, umfassend:
a) Salicyloyl-Sphingoidbasen und/oder Derivate davon, und
b) einen dermatologisch zulässigen Träger
zum Behandeln und/oder Vorbeugen von Cellulitis durch Steigern der Hautstrukturproteinmengen (Collagen und Fibrillin) und Reduzieren der MMP-1-Spiegel.

2. Verwendung einer Kosmetik-Zusammensetzung nach Anspruch 1, wobei es sich bei den Salicyloyl-Sphingoidbasen um Salicyloyl-Phytosphingosin handelt.

3. Verwendung einer Kosmetik-Zusammensetzung nach Anspruch 1, wobei es sich bei den Salicyloyl-Sphingoidbasen um Salicyloyl-Sphingosin, Salicyloyl-Sphinganin oder Salicyloyl-6-hydroxysphinganin handelt.

4. Verwendung einer Kosmetik-Zusammensetzung nach Anspruch 1 bis 3, wobei eine oder mehrere Hydroxylgruppen der Sphingoidbase-Einheit der Salicyloyl-Sphingoidbase als organische und anorganische Ester modifiziert ist/sind.

5. Verwendung einer Kosmetik-Zusammensetzung nach Anspruch 1 bis 3, wobei eine oder mehrere Hydroxylgruppen der Sphingoidbase-Einheit der Salicyloyl-Sphingoidbase durch Glycosylierung modifiziert ist.

6. Verwendung einer Zusammensetzung nach Anspruch 1 bis 5, umfassend 0,001 bis 20Gew.-%, vorzugsweise 0,01 bis 10 Gew.-% und am stärksten bevorzugt 0,02 bis 2 Gew.-% der Zusammensetzung aus Salicyloyl-Sphingoidbase, Sphingoidbase und/oder den Derivaten.

7. Verwendung einer topischen Zusammensetzung nach den Ansprüchen 1 bis 6 in Kombination mit mindestens einer Verbindung, ausgewählt aus der Gruppe, bestehend aus:
a) Sphingoid- und Phospholipid-Derivaten: Ceramiden, Phytosphingosin, Sphingosin, Pseudoceramiden, Phospholipiden, Lysophospholipiden,
b) Antioxidationsmitteln und Vitaminen, wie Tocopherol und Derivaten, Ascorbinsäure und Derivaten, Niacinamid und Derivaten, Vitamin-Komplexen, Alpha-Liponsäure, Retinol und Derivaten, Panthenol,
c) entzündungshemmenden Mitteln, wie Bisabolol, Allantoin, Phytantriol, Coenzym Q10, Idebenon,
d) Pflanzenstoffen, wie Polyphenolen, Flavonoiden und Isoflavonen,
e) Feuchtigkeitsspendern, wie Aminosäuren, Hyaluronsäure und Derivaten, Kreatin und Derivaten, Trimethylglycin, Myoinositol, Pyroglutaminsäure und Derivaten, Taurin, Guanidin und Derivaten und Hydroxysäuren,
f) Hautaufhellungsmitteln, wie Kojisäure, Arbutin, Vitamin C und Derivaten, Hydrochinon,
g) Peptiden, modifizierten Peptiden, Proteinhydrolysaten,
h) Koffein,
i) Sonnenschutzmitteln und UV-Absorptionsmitteln.

## Revendications

1. Utilisation d'une composition cosmétique comprenant :
a) une base salicyloyl-sphingoïde et/ou ses dérivés, et
b) un véhicule acceptable sur le plan dermatologique
pour traiter et/ou prévenir la peau cellulitique en augmentant le niveau des protéines de la structure du derme (collagène et fibrilline) et en réduisant les niveaux de MMP-1.

2. Utilisation d'une composition cosmétique selon la revendication 1 dans laquelle la base salicyloyl-sphingoïde est la salicyloyl-phytosphingosine.

3. Utilisation d'une composition cosmétique selon la revendication 1 dans laquelle la base salicyloyl-sphingoïde est la salicyloyl-sphingosine, la salicyloyl-sphinganine ou la salicyloyl-6-hydroxysphinganine.

4. Utilisation d'une composition cosmétique selon les revendications 1 à 3 dans laquelle un ou plusieurs des groupes hydroxyle du reste base sphingoïde de la base salicyloyl-sphingoïde sont modifiés en esters organiques et inorganiques.

5. Utilisation d'une composition cosmétique selon les revendications 1 à 3 dans laquelle un ou plusieurs des groupes hydroxyle du reste base sphingoïde de la base salicyloyl-sphingoïde sont modifiés par glycosylation.

6. Utilisation d'une composition selon la revendication 1 à 5 comprenant 0,001 à 20%, de préférence 0,01 à 10% en poids et tout particulièrement 0,02 - 2%, en poids de la composition, de ladite base salicyloyl-sphingoïde, de ladite base sphingoïde et/ou de leurs dérivés.

7. Utilisation d'une composition à usage local selon les revendications 1 à 6 en association avec au moins un composé choisi dans le groupe constitué par
a) les dérivés de sphingoïde et de phospholipides : céramides, phytosphingosine, sphingosine, pseudocéramides, phospholipides, lysophospholipides,
b) les antioxydants et les vitamines tels que le tocophérol et ses dérivés, l'acide ascorbique et ses dérivés, le niacinamide et ses dérivés, les complexes vitaminiques, l'acide alpha-lipoïque, le rétinol et ses dérivés, le panthénol,
c) les anti-inflammatoires tels que bisabolol, allantoïne, phytantriol, Coenzyme Q10, Idébénone,
d) les agents botaniques tels que les polyphénoliques, les flavonoïdes et les isoflavones,
e) les agents hydratants tels que les acides aminés, l'acide hyaluronique et ses dérivés, la créatine et ses dérivés, la triméthylglycine, le myoinositol, l'acide pyroglutamique et ses dérivés, la taurine, la guanidine et ses dérivés, et les hydroxyacides,
f) les agents de blanchiment de la peau tels que l'acide kojique, l'arbutine, la vitamine C et ses dérivés, l'hydroquinone,
f) les peptides, les peptides modifiés, les hydrolysats de protéines,
h) la caféine,
i) les écrans solaires et les absorbeurs UV.
